# EUROPEAN PATENT SPECIFICATION

(11) **EP 2 311 323 B1**
(45) Date of publication and mention of the grant of the patent: **16.01.2013**
(21) Application number: 09013171.5
(22) Date of filing: 19.10.2009
(51) Int. Cl.: C12N 9/52, A61K 38/48, A61L 2/18, A01N 63/02, A01P 1/00

(54) **Methods for degradation of protein deposits and prions**
Abbaumethoden von Proteinablagerungen und Prionen
Méthodes pour la dégradation des dépôts de protéine et prions

(43) Date of publication of application: 20.04.2011
(73) Proprietor: Univerza v Ljubljani, 1000 Ljubljana (SI)
(72) Inventor: Poklar Ulrih, Natasa, 1000 Ljubljana (SI); Vilfan, Tanja, 1000 Ljubljana (SI)
(74) Representative: Schmidt, Karsten

(56) References cited:
- WO-A2-02/053723
- US-A1- 2004 091 474
- US-A1- 2009 104 073
- MCLEOD A H ET AL: "Proteolytic inactivation of the bovine spongiform encephalopathy agent" BIOCHEMICAL AND BIOPHYSICAL RESEARCH COMMUNICATIONS, ACADEMIC PRESS INC. ORLANDO, FL, US, vol. 317, no. 4, 14 May 2004 (2004-05-14), pages 1165-1170, XP004502892 ISSN: 0006-291X

## Description

The present invention relates to ex vivo methods for protein and/or prion protein degradation. Protein and/or prion protein is degraded with extracellular proteolytic enzymes of hyperthermophilic *Aeropyrum pernix* K1.

### Background of the invention

Prion diseases are transmissible spongiform encephalopathies (TSE), a group of neurodegenerative diseases that affect the central nervous system. They have a very long incubation period, there has not been a therapy found against this group of disorders; thus, they are always terminal. Prion diseases may develop sporadically, for genetic reasons or after an infection with prion proteins. They have been described in humans and animals/mammals, i.e. sheep and cattle. Among the most extensively studied TSEs are scrapie in sheep, goats and experimental animals, BSE in cattle, kuru and Creutzfeldt-Jakob disease (CJD) in human.

It is believed that TSEs are caused by prions (Griffith 1967, Prusiner 1982) which are proteinaceous infectious protein particles that lack nucleic acids (Prusiner 1982). The infectious form of the prion protein, PrP^{Sc} is generated from the normal cellular form of the protein, PrP^{c}. The PrP^{c} form is encoded in a genome and is synthesised in cells of different types; nevertheless, the level of its expression is the highest in the central nervous system cells. Each form of the protein has a specific tertiary structure. Whereas the PrP^{c} form mostly contains alpha-helixes, the PrP^{Sc} form has a higher level of beta-structures. The infectious form of the protein develops in a post-translational process, during which a conformational change causes the transformation of the PrP^{c} form into the PrP^{Sc} form, the prion protein (Bolton et al. 1984, Borchelt et al. 1990). The consequence of different tertiary structures of the both protein forms are also different physico-chemical characteristics. Because of the tertiary structure that is reach in beta-structure, the PrP^{Sc} form is highly resistant to a degradation with proteolytic enzymes and insoluble in solutions of detergents, while the PrP^{c} form can readily be digested by proteolytic enzymes and is soluble in solutions of detergents.

Prion proteins and prion-related diseases have been in the focus of scientific research since the mid-1980's, when the first case of the bovine spongiform encephalopathy, BSE, occurred in the United Kingdom. In the same country the first case of such illness was described in human and was classified as variant Creutzfeldt-Jakob disease, vCJD. It is believed that vCJD is transmitted from the cattle to human via food, and also maybe vaccines and drugs containing bovine proteins as stabilizing agents. Additionally, it was also confirmed that the disease can be transmitted by blood transfusions of asymptomatic donors, which makes a basic and applicative research in the field of prion proteins more and more important.

One of the means of TSE transmission is surgical material and instruments, that are infected during operations. The infected equipment then represents a constant threat since the physico-chemical characteristics of prion proteins such as resistance against high temperatures and aggressive detergents make it impossible to sterilize such equipment with standard procedures of sterilization.

All of the above gives grounds for the need of a new, efficient, environmentally friendly way of inactivation, elimination and/or degradation of prion protein.

In the recent years several articles and patents were published, describing the proteolytic degradation of prion protein. The majority of the cases describe procedures that first include degradation of prion protein with proteolytic enzymes and then additional chemical or physical treatment, to obtain an almost complete degradation of the PrP^{Sc} form. Degradation with commercially available proteolytic enzymes was coupled with treatment in 0.1 M NaOH or 2 % SDS (Kasermann and Kempf 2003, Jackson 2005). Degradation of BSE and scrapie homogenates with *Bacillus licheniformis* keratinase PWD-I was coupled with incubation at temperatures exceeding 100°C (Langeveld 2003). The capability of some proteolytic enzymes to digest the PrP^{Sc} form was confirmed by western blot analysis (Mitsuki et al. 2006; Müller-Hellwig at al. 2006, Yoshioka et al. 2007). There are no data available about the remaining infectivity of the obtained degradation products.

US 6720355 discloses a method of the prion protein deactivation by heating in the presence of sodium dodecyl sulphate (SDS).

US 6719988 discloses a method of the prion protein deactivation with heating in the presence of alkylsulphates at pH 2.5 to 4.5.

US 6743899 discloses a method of the prion protein deactivation in alkaline solution, pH 10 to 13.

WO 2007/023178 discloses a method of the prion protein deactivation with lipophilic substances in samples containing bovine fat and bones that are used in production of soaps, detergents, cosmetics and animal food.

WO 2004/039418 discloses a method of the prion protein deactivation on surgical instruments with sodium dodecyl sulphate (SDS) detergent combined with several proteolytic enzymes (proteinase K, papain, pronase and bromelanin).

US 6613505 discloses a method of the prion protein deactivation with PWD-I keratinase isolated from *Bacillus licheniformis.* The biological material is heated to 150°C during the procedure.

US 2006/12739 discloses a method of the prion protein deactivation with subtilysin proteinase at 40°C.

US 5756678 and EP 0742018 disclose a method of the prion protein deactivation in connective tissue with sodium hydroxide.

US 4352625 discloses a method of the prion protein deactivation on surfaces with ultrasound.

US 6707254 discloses a low temperature plasma sterilizing system and method.

WO 2003/077835 discloses compositions of sodium dodecyl sulphate (SDS) solutions for prion protein degradation.

US 6322802 discloses sterilization of equipment with standard sterilization techniques and polycationic dendrimers.

US 7303907 discloses a methods of protein deactivation with thermo-resistant proteolytic enzymes from various microorganisms. Experiments applying thermostable proteolytic enzymes of *A. pernix* (pernisine) for protein degradation are mentioned. The proteolytic enzyme is purified using ammonium sulphate precipitation and dye-ligand chromatography prior to its use in protein degradation. No data on the successful application of *A. pernix* proteases for the degradation of prion protein is presented in US 7303907. In a subsequent publication from the same authors (McLeod et al, 2004), it is shown that proteolytic enzymes of *A. pernix* did not degrade prion protein in their experiments. Without wishing to be bound by theory, it is assumed that the inability of the proteolytic enzyme of *A. pernix* in US 7303907 results from the fact that the enzyme is purified and thereby separated from other essential components of the culture supernatant. The purification step separates the proteolytic enzymes from other components of the *A. pernix* culture supernatant, which appear to be essential for the prion-degrading activity of the *A. pernix* serine protease (pernisine). US 7303907 thus does not teach how to use proteolytic enzymes of *A. pernix* for the degradation of prion proteins.

The majority of described procedures include treatment of samples at extreme conditions, e.g. high pressure, high temperature, extremely acidic or alkaline pH, addition of aggressive substances (alkylsulphates). The application of high pressure, extremely acidic or alkaline pH, addition of aggressive substances (alkylsulphates) complicates the process and leads to increased cost.

*Aeropyrum pernix* K1 (JCM 9820) is a Gram negative, heterotrophic, strictly aerobic, hyperthermophilic marine archea. It was isolated from a solphataric vent on Kodakara-Jima island in Japan in 1993 (Sako et al. 1996). The complete genome of *A. pernix* was sequenced (Kawarabayasi et al. 1999). The optimal growth of *A. pernix* with doubling time of 200 min is achieved at temperatures between 90°C and 95°C, neutral pH and 3.5% salinity.

### Summary of the invention

In view of the above state of the art it is an object of the present invention to provider ex vivo methods for the effective degradation of protein and/or prion protein.

Accordingly, the present invention relates to the ex vivo use of *A*. *pernix* serine protease for the degradation of protein and/or prion protein. Preferably, the invention relates to the degradation of protein deposits (or protein aggregates) and/or prion protein. In a preferred embodiment of the invention, the *A. pernix* serine protease is in the form of a filtrate obtained by filtration of an *A. pernix* culture supernatant, preferably obtained with microfiltration through a filter having a cut-off of equal to or larger than 30 kDa (henceforth, the "R30 fraction").

The invention thus relates to the ex vivo use of an *A*. *pernix* culture supernatant comprising: *A. pernix* serine protease, or an active fragment thereof, or a protein at least 50, 60, 80, 90, 95, 97, 99, or 99.9% identical to *A. pernix* serine protease (SEQ ID NO:1), or an active fragment thereof, for the degradation of protein and/or prion protein.

In a preferred embodiment of the invention the *A. pernix* is *A. pernix* K1.

In a preferred embodiment of the invention said *A. pernix* culture supernatant is obtained by obtained from ultra-filtration of *A. pernix* culture supernatant with a cut-off molecular weight of at least 30 kDa.

In another preferred embodiment of the invention said degradation of said protein and/or prion protein is for the sterilization of surgical equipment. In another preferred embodiment of the invention, said degradation of said protein and/or prion protein is for the cleaning of textiles. In another preferred embodiment of the invention, said degradation of said protein and/or prion protein is in food.

The invention further relates to an ex vivo method of protein and/or prion protein degradation comprising incubation of the protein and/or prion protein with an *A. pernix* culture supernatant comprising *A. pernix* serine protease, or an active fragment thereof, or a protein at least 80% identical to *A. pernix* serine protease, or an active fragment thereof.

In a preferred embodiment of the invention, said *A. pernix* culture supernatant is in form of a filtrate obtained from ultra-filtration of *A. pernix* culture supernatant with a cut-off molecular weight of at least 30 kDa.

In another preferred embodiment of the invention, said method is for the treatment of protein and/or prion infected waste, or for the cleaning of protein and/or prion-infected textiles, or is in food. In another preferred embodiment of the invention, said protein and/or prion protein degradation is on the surface of a solid material.

Further embodiments of the invention are described in the detailed description of the invention and in the appended claims.

### Short description of the Figures

Figure 1 shows the degradation of prion protein after addition of R30 fraction according to the invention.
Figure 2 shows the degradation of different isoforms of the PrP^{Sc} protein.
Figure 3 shows the detection of peptide fragments after PrP^{Sc} degradation.
Figure 4 shows the detection of PrP^{Sc} after precipitation with phosphotungstic acid.

### Detailed description of the invention

*"A. pernix* serine proteinase", within the meaning of the invention shall be understood as being a protein having the amino acid sequence of SEQ ID NO:1, or a protein sequence 80, 85, 90, 95, 97, 98, 99, or 99.9% identity with SEQ ID NO:1. Alternatively, *"A. pernix* serine proteinase" shall be understood to relate to the protein recoded in public database UniProtKB (http://www.uniprot.org/) under ID Q9YFI3_AERPE. Alternative, *"A. pernix* serine proteinase" relates to a protein encoded by DNA which hybridizes under stringent conditions to DNA encoding SEQ ID NO:1.

"Active fragments" of a protein, according to the invention, shall be understood as being fragments of said protein having the same biochemical activity as the original protein. Fragments of a protein, according to the invention, are preferably fragments of 10%, 20%, 30%, 40%, 50%, 60%, 70%, 80%, 90%, 95%, 98%, 99% of the original length.

The expression "prion protein" shall be understood as relating to the PrP^{SC} and/or to the PrP^{c} protein, generally known in the art.

"Culture supernatant", within the context of the present invention, shall be understood any liquid medium used for cultivation of a microbial organism (e.g. *A. pernix*) after cultivation of the microbial organism has taken place. Culture supernatants of the present invention may also comprise disintegrated cell mass, if the cells of the microbial organism were disintegrated after cultivation. A culture supernatant, according to the invention, preferably comprises extracellular proteins of said organism, which are excreted upon cultivation. A culture supernatant, according to the invention, may comprise intracellular proteins of the organism, in particular, if at least part of the cells of the organism disintegrated during cultivation, or thereafter, e.g. by suitable disintegration techniques.

"Degradation" of a protein, within the meaning of the present invention shall be understood to be the degradation of the protein into parts or fragments or amino acids of the protein, wherein 20%, preferably 50%, 75%, 85%, 90%, 95%, 98%, 99%, 99.9%, most preferably 100% of the original protein are degraded.

A "protein deposit", according to the invention, shall be understood to be a local agglomeration of protein in un-dissolved form on the surface of a solid object.

An "R30 fraction", within the meaning of the invention, shall be understood as being the filtrate of a culture supernatant filtered trough a filter having a cut-off molecular weight of at least 30 kDA. In other preferred embodiments of the invention, the cut-off molecular weight of the filter is 10, 20, 40, 50, 100, 200, 400, or 800 kDa.

"Surgical equipment" shall have the ordinary meaning of this term as it is understood in the art. Surgical equipment comprises *i.a*. scissors, forceps, needles, needle holders, retractors, scalpels, lancets, endoscopes, towel clamps, drills, as well as other surgical equipment.

Within the context of the present invention, the "% identity" of a protein relative to a reference protein of a defined length shall be understood as follows: A peptide that is 50 percent identical to a reference polypeptide that is 100 amino acids long can be a 50 amino acid polypeptide that is completely identical to a 50 amino acid long portion of the reference polypeptide. It might also be a 100 amino acid long polypeptide, which is 50 percent identical to the reference polypeptide over its entire length. Of course, other polypeptides will meet the same criteria. The term "sequence identity" as used herein means that the sequences are compared as follows. The sequences are aligned using Version 9 of the Genetic Computing Group's GAP (global alignment program), using the default (BLOSUM62) matrix (values -4 to +11) with a gap open penalty of -12 (for the first null of a gap) and a gap extension penalty of -4 (per each additional consecutive null in the gap). After alignment, percentage identity is calculated by expressing the number of matches as a percentage of the number of amino acids in the reference polypeptide.

"Stringent conditions", within the meaning of the invention, shall be understood as being the stringent conditions as set forth according to Sambrook et al. (2^{nd} ed., 1989, Cold Spring Harbor Laboratory Press, Cold Spring Harbor, N.Y.)

*A. pernix* excretes extracellular proteases into the growth medium. An *A. pernix* serine protease preparation according to the invention can be obtained as follows:
*A. pernix* is cultivated until the stationary growth phase. The cells are removed from the growth medium by filtration and different protein fractions with respect to protein molecular weight are prepared by ultra-filtration. Extracellular proteolytic enzymes, e.g. those in the R30 fraction, degrade protein and/or prion proteins in solutions with neutral pH without prior chemical or physical treatment of the protein and/or prion containing samples. R30 fraction is obtained by ultra-filtration through a filter with a cut-off molecular weight of 30 kDa. The fraction contains partly isolated proteolytic enzyme of *A. pernix* which has an optimal activity at 80°C. In addition, the tests with protease inhibitors, MALDI- TOF MS and analysis of nucleotide and amino acid sequences of *A. pernix* revealed that R30 fraction contained only one proteolytic enzyme, namely serine protease pernisine, and fragments of various ABC transporters.

Treatment of samples of brain homogenates affected with prion proteins (BSE, vCJD, protein deposits of Alzheimer and Parkinson's diseases, etc.) completely eliminates the prion proteins from the samples. It was shown that protinase K is not as effective. The presence of prion proteins and prion protein fragments in samples after treatment with proteolytic enzymes was monitored with known immunological methods (western blot, dot blot, precipitation with phosphotungstic acid). Detection was done with different monoclonal antibodies against PrP^{c} and PrP^{Sc} forms.

Growth conditions which support an appropriate biomass gain were optimized in our laboratory (Milek et al. 2005). The research was then focused to different protein groups of the microorganism, e.g. DNA-binding proteins and proteolytic enzymes which was made easier when the genome was published (Kawarabayasi et al. 1999). Since the *A. pernix* proteins have very high temperature optima (they retain at least some of their activity at temperatures even higher than 100°C), there has been a growing interest in proteins of this microorganism ever since the genome discovery.

The main source of nutrients of hyperthermophilic *A. pernix* are proteins. Based on that, it was speculated the archea can be a source of variety of proteolytic enzymes that would be capable of prion protein degradation. Although proteolytic enzymes are indispensible in a life of every cell, only three proteolytic enzymes were identified so far from *A*. *pernix*; intracellular proteinase pemilase (Chavez Croocker et al. 1999), extracellular proteinase aeropirolysin (Sako et al. 1997) in recombinant extracellular proteinase pernisine (Catara et al. 2003).

Proteolytic enzymes isolated from *A. pernix* and their recombinant analogues may be applied not only for protein and/or prion protein degradation in waste, but also in many other procedures, such as sterilization of surgical equipment, to clean and/or remove the protein deposits (films) from solid surfaces as additions to washing powder, for complete protein hydrolysis in food industry (removal of undesired peptides with bitter taste, allergens etc.), and as a substitute of proteinase K in such processes.

Advantages of proteolytic reactions according to the invention are: substrates can be used in higher concentrations, reaction rates are higher, lower risk of infections with other temperature-sensitive material.

Proteolytic enzymes of *Aeropyrum pernix* were shown to be extremely effective in degradation of the protein and/or prion proteins in solutions. Proteolytic enzymes in R30 protein fraction degrade protein and/or prion aggregates very quickly (in less than 10 min). Furthermore, their effectiveness is not lowered even if the substrate concentration is increased up to by 4-fold.

The present invention thus relates to the ex vivo use of A. *pernix* serine protease (pernisine) in a culture supernatant of, or in an R30 fraction of, *A. pernix* for protein and/or prion protein degradation.

In a preferred embodiment of the invention, said *A. pernix* serine protease in a culture supernatant of, or in an R30 fraction of, *A. pernix* is obtained by ultra-filtration of *A. pernix* culture supernatant through a filter with a cut-off molecular weight of equal to or larger than 30 kDa.

Without being bound by theory, it is understood that ultra-filtration with a cut-off at 30 kDa yields both the *A. pernix* serine protease and further components of the original supernatant, such as fragments of various ABC transporters, in the filtrate. A high activity of the *A. pernix* serine protease is obtained in the R30 fraction. This is a very surprising, since it was shown that a highly purified *A. pernix* serine protease preparation was proteolytically inactive (see McLeod et al., 2004).

Another aspect of the invention is the use of methods of the invention for sterilization of surgical equipment.

Another aspect of the invention is protein degradation for the cleaning of textiles. Preferably, compositions of the inventions are added to washing powder. This allows protein and/or prion protein to be removed from textiles during the washing procedure.

Another aspect of the invention is the complete protein hydrolysis in food.

Protein and/or prion protein degradation, according to the invention, preferably takes place at elevated temperatures above 50°C, 60°C, 70°C, 75%, most preferably 80°C. Protein and/or prion protein degradation, according to the invention, preferably takes place at temperatures equal to or below 121°C, 110°C, 100°C, 90°C, 85°C, or 80°C. Preferred temperature ranges are between 50°C and 121°C, preferably between 70°C and 100°C, or between 75°C and 85°C.

Protein and/or prion protein degradation, according to the invention, preferably takes place at pH above 5, preferably above 6, 7, 7.5, most preferably above pH 8. Protein and/or prion protein degradation, according to the invention, preferably takes place at a pH below 10, 9.5, 9, 8.7 most preferably below pH 8.6. Preferred pH ranges are between 5 and 10, preferably between 6 and 9.6, between 7 and 9, between 7.5 and 8.7, most preferred between 8.4 and 8.6.

The invention further relates to ex vivo methods of protein and/or prion protein degradation comprising incubation of the protein with *A. pernix* serine protease in an *A. pernix* culture supernatant or R30 fraction thereof. Preferably the *A. pernix* serine protease in an *A. pernix* culture supernatant, or R30 fraction thereof is added to a composition containing the protein to be degraded.

Protein degradation according to the invention can be the degradation of soluble protein, as well as the degradation of protein deposits. In a preferred embodiment of the invention, protein degradation is an ex vivo degradation of protein deposits.

### Examples

### Example 1

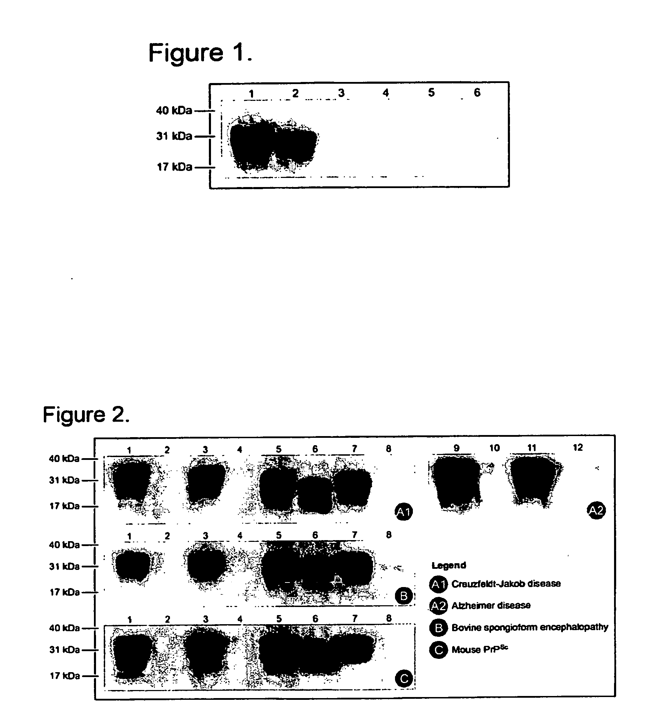

In Figure 1 is shown the degradation of prion protein PrP^{Sc} after addition of R30 fraction in human brain homogenate affected with Creutzfeldt-Jakob disease with temperature and time. Supernatants of infected human brain homogenate were incubated for one hour at 92°C without R30 fraction (lane I) or with R30 fraction for one hour at 25°C (lane 2); 10 min at 92°C (lane 3); 20 min at 92°C (lane 4); 40 min at 92°C (lane 5); and one hour at 92°C (lane 6). Proteins were separated and transferred onto a nitrocellulose membrane. Immuno-reactive components were detected with 6H4 monoclonal antibodies.

### Example 2

Figure 2 shows the degradation of different isoforms of prion protein PrP^{Sc} with R30 fraction. Different brain homogenates were used in each experiment: unaffected human brain homogenate (panel A1: lanes 1 to 4), unaffected bovine brain homogenate (panel B: lanes 1 to 4) or unaffected mouse brain homogenate (panel C: lanes 1 to 4) and affected human brain homogenate (panel A1: lanes 5 to 8; panel A2; lanes 9 to 12), affected bovine brain homogenate (panel B; lanes 5 to 8) or affected mouse brain homogenate (panel C; lanes 5 to 8). Supernatants of brain homogenates were incubated without (lanes 1, 5, 9) or with 0.75 µg of proteinase K (lanes 2, 6, 10) for 20 min at 37°C; without (lanes 3, 7, 11) or with (lanes 4, 8, 12) R30 fraction for 20 min at 92°C. Proteins were separated and transferred onto a nitrocellulose membrane. Immuno-reactive components were detected with 6H4 monoclonal antibodies.

### Example 3

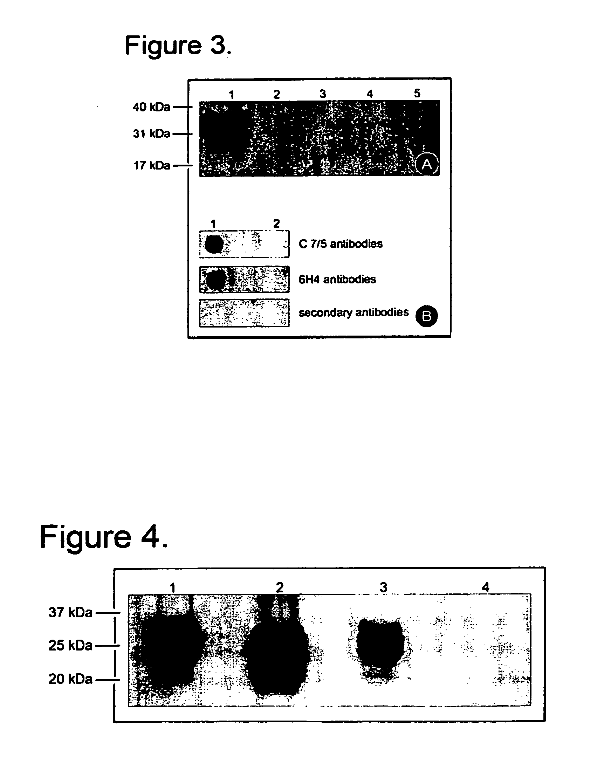

Figure 3 shows the detection of peptide fragments after prion protein PrP^{Sc} degradation. Panel A: Supernatants of unaffected human brain homogenate incubated for 20 min at 92°C (lane 1); reaction mixtures with 3 µl, 6 µl, 9 µl or 12 µl of supernatant of human brain homogenate affected with Creutzfeldt-Jakob disease (lanes 2 to 5) were incubated with R30 fraction for 20 min at 92°C. Proteins were separated and transferred onto a nitrocellulose membrane. Immuno-reactive components were detected with 6H4 monoclonal antibodies. Panel B: 20 times diluted supernatant of human brain homogenate affected with Creutzfeldt-Jakob disease was incubated without (lane 1) or with R30 fraction (lane 2) for 20 min at 92 °C. Proteins were dot-blotted onto a nitrocellulose membrane and immuno-reactive components were detected with different monoclonal antibodies, as indicated.

### Example 4

Figure 4 shows the detection of prion protein PrP^{Sc} after precipitation with phosphotungstic acid (NaPTA). Supernatants of human brain homogenate affected with Creutzfeldt-Jakob disease were incubated for 20 min at 37°C without (lane 1) or with (lane 2) proteinase K and then precipitated with NaPTA. Supernatants of human brain homogenate affected with Creutzfeldt-Jakob disease were incubated for 20 min at 92°C without (lane 3) or with R30 fraction (lane 4) and then precipitated with NaPTA. Proteins were separated and transferred onto a nitrocellulose membrane. Immuno-reactive components were detected with 6H4 monoclonal antibodies.

### Example 5

*A. pernix* (the full microorganism) is used for protein degradation. *A. pernix* is grown in a bioreactor with protein waste as an energy source. Detection of the prion protein and its degradation products is performed with commercially available specific monoclonal antibodies, using known techniques, such as ELISA, western blot and dot blot.

### Example 6

Preparation and analysis of the R30 fraction of a culture supernatant.

Cultivation of *A. pernix* was stopped after 40 h of growth and cells were removed by centrifugation at 10000 g and filtration through 45 µm and 20 µm cellulose nitrate filters. The growth medium was then concentrated by ultra-filtration using YM30 membranes (Millipore, USA). Concentrated growth medium was separated on a gel filtration column with a Sephadex G100 equilibrated with PBS buffer. The fractions with the highest specific proteolytic activity were subjected to an additional step of ultra-filtration. The R30 fraction gained in the final step of ultra-filtration was used in the following experiments.

For protease content analysis, the R30 fraction was separated using a standard SDS-PAGE procedure. After separation on a 10 % SDS-polyacrylamid gel the proteins were stained using standard techniques and selected protein bands were analyzed by MALDI-TOF MS at Aberdeen Proteomics (University of Aberdeen, Great Britain).

### Literature

Bolton, D.C., McKinley M.P., Prusiner, S.B.(1984) Molecular characteristics of the major scrapie prion protein. Biochemistry 23,5898 - 5906.
Borchelt, D.R., Scott, M., Taraboulos, A., Stahl, N., Prusiner, S.B. (1990)Scrapie and cellular prion proteins differ in their kinetics of synthesis and topology in cultured cells. J Cell Bio. 110,743 - 752.
Catara, G., Ruggiero, G., La Cara, F., Digilio, F.A., Capasso, A., and Rossi, M. (2003). A novel extracellular subtilisin-like protease from the hyperthermophile Aeropyrum pernix K1 biochemical properties, cloning, and expression. Extremophiles 7, 391 - 399.
Chavez Croocker, P., Sako, Y., and Uchida, A. (1999). Purification and characterization of an intracellular heat-stable proteinase (pernilase) from the marine hyperthermophilic archaeon Aeropyrum pernix K1 Extremophiles 3,3-9.
Griffith, J.S. (1967) Self -replication and scrapie. Nature 215, 1043 - 1044.
Jackson, G. S., McKintosh, E., Flechsig, E., Prodromidou, K., Hirsch, P., Linehan, J., Brandner, S., Clarke, A.R., Weissmann, C., and Collinge, J. (2005). An enzyme-detergent method for effective prion decontamination of surgical steel. J. Gen. Virol. 86,869 - 878.
Kasermann, F. and C. Kempf (2003). Sodium hydroxide renders the prion protein PrPSc sensitive to proteinase K. J. Gen. Virol. 84, 3173 - 3176.
Kawarabayasi, Y., Hino, Y., Horikawa, H., Yamazaki, S., Haikawa, Y., Jin-no, K., Iakahashi, M., Sekine, M., Baba, S., Ankai, A., Kosugi, H., Hosoyama, A., Fukui, S., Nagai, Y., Nishijima, K., Nakazawa, H., Iakamiya, M., Masuda, S., Funahashi, T., Ianaka, I., Kudoh, Y., Yamazaki, J., Kushida,N., Oguchi, A., Aoki, K., Kubota, K., Nakamura, Y., Nomura, N., Sako, Y., Kikuchi, H. (1999). Complete genome sequence of an aerobic hyperthermophilic crenarchaeon, Aeropyrum pernix K1. DNA Res. 6, 83-101.
Langeveld, J.P., Wang, J.J., Van de Wiel, D.F., Shih, G.C., Garssen, G.J., Bossers, A., and Shih, J.C. (2003). Enzymatic digestion of prion protein in brain stem from infected cattle and sheep. J. Inf. Dis. 188, 1782 - 1789.
McLeod et al. (2004), Biochemical and Biophysical Research Communications, 317, 1165-1170
Milek, I., Skrt, M., Cigic, B., Kaletunc, G., Poklar Ulrih, N. (2005). Optimization of growth for the hyperthermophilic archaeon Aeropyrum pernix on small-batch scale. Can. J. Microbiol. 51, 805 - 809.
Mitsuiki, S., Hui, Z., Matsumoto, D., Sakai, M., Moriyama, Y. Furnkawa, K., Kanouchi H., and Oka, T. (2006). Degradation of PrPSc by keratinolytic protease from Nocardiopsis sp. TOA-1. Biosci. Biotechnol. Biochem. 70, 1246 - 1248.
Müller-Hellwig, S., Groschup, M.H., Pichner, R., Gareis, M., Martlbauer,E., Scherer, S., and Lessner, M.J. (2006). Biochemical evidence for the proteolytic degradation of infectious prion protein PrPSc in hamster brain homogenates by foodborne bacteria. Syst. Appl. Microbiol. 29, 165 - 171.
Prusiner, S.B. Novel proteinaceous infectious particles cause scrapie. Science 216, 136 - 144 (1982).
Sako, Y., Nomura, N., Uchida, A., Ishida, Y., Morii, H., Koga, Y., Hoaki, T., Maruyama, T. (1996). Aeropyrum pernix gen. nov., sp. nov., a novel aerobic hyperthermophilic archaeon growing at temperatures up to 100°C. Int. J. Syst. Bacteriol. 46, 1070-1077.
Sako, Y., P. Chavez Croocker and Ishida,Y. (1997). An extremely heat-stable extracellular proteinase (aeropyrolysin) from the hyperthermophilic archaeon Aeropyrum pernix K1. FEBS Lett. 415, 329 - 334.
Telling, G.C (2000). Prion protein genes and prion diseases: studies in transgenic mice. Review. Neuropathol. and. Appl. Neurobiol. 26, 209 - 220.
Yoshioka, M., Miwa, T., Horii, H., Iakata, M., Yokoyama, T., Nishizawa, K., Watanabe, M., Shinagawa, M., and Murayama, Y.(2007). Characterization of a proteolytic enzyme derived from a Bacillus strain that effectively degrades prion protein. J. Appl. Microbiol. 102,509 - 515.

## Claims

1. An ex vivo *u*se of an *A. pernix* culture supernatant comprising *A. pernix* serine protease, or an active fragment thereof, or a protein at least 80% identical to *A. pernix* serine protease, or an active fragment thereof, for the degradation of prion protein and/or protein deposits.

2. Use of claim 1, wherein said *A. pernix* culture supernatant is purified by ultra-filtration with a cut-off molecular weight of at least 30 kDa.

3. Use of any one of the above claims, wherein said degradation of said prion protein and/or protein deposits is in the sterilization of surgical equipment.

4. Use of any one of the above claims, wherein said degradation of said prion protein and/or protein deposits is in the cleaning of textiles.

5. Use of any one of the above claims, wherein said degradation of said prion protein and/or protein deposits is in food.

6. An ex vivo method of prion protein and/or protein deposits degradation comprising incubation of the prion protein and/or protein deposits with an *A. pernix* culture supernatant comprising *A. pernix* serine protease, or an active fragment thereof, or a protein at least 80% identical to *A. pernix* serine protease, or an active fragment thereof.

7. A method of claim 6, wherein said *A. pernix* culture supernatant is in form of a filtrate obtained from ultra-filtration of *A. pernix* culture supernatant with a cut-off molecular weight of at least 30 kDa.

8. Method of any one of claims 6 and 7, wherein said method is for the treatment of protein and/or prion infected waste.

9. Method of any one of claims 6-8, wherein said method is for the cleaning of protein and/or prion-infected textiles.

10. Method of any one of claims 6-9, wherein said prion protein degradation and/or protein deposit degradation is in food.

11. Method of any one of claims 6-10, wherein said prion protein degradation and/or protein deposits degradation is on the surface of a solid material.

## Patentansprüche

1. Ex-vivo-Verwendung eines Überstands einer Kultur von *A*. *pernix,* umfassend eine Serinprotease von *A*. *pernix* oder ein aktives Fragment davon oder ein Protein, das zu mindestens 80% mit einer Serinprotease von *A*. *pernix* identisch ist, oder ein aktives Fragment davon, für den Abbau von Prionenprotein und/oder Proteinablagerungen.

2. Verwendung nach Anspruch 1, wobei der Überstand einer Kultur von *A. pernix* durch Ultrafiltration mit einem Ausschluss-Molekulargewicht von mindestens 30 kDa gereinigt wird.

3. Verwendung nach einem der vorherigen Ansprüche, wobei der Abbau des Prionenproteins und/oder der Proteinablagerungen bei der Sterilisation chirurgischer Instrumente erfolgt.

4. Verwendung nach einem der vorherigen Ansprüche, wobei der Abbau des Prionenproteins und/oder der Proteinablagerungen bei der Reinigung von Textilien erfolgt.

5. Verwendung nach einem der vorherigen Ansprüche, wobei der Abbau des Prionenproteins und/oder der Proteinablagerungen in Lebensmitteln erfolgt.

6. Ex-vivo-Verfahren für den Abbau von Prionenprotein und/oder Proteinablagerungen, umfassend die Inkubation des Prionenproteins und/oder der Proteinablagerungen mit einem Überstand einer Kultur von *A*. *pernix*, umfassend eine Serinprotease von *A*. *pernix* oder ein aktives Fragment davon oder ein Protein, das zu mindestens 80 % mit einer Serinprotease von *A. pernix* identisch ist, oder ein aktives Fragment davon.

7. Verfahren nach Anspruch 6, wobei der Überstand einer Kultur von *A*. *pernix* in Form eines durch Ultrafiltration eines Überstands einer Kultur von *A. pernix* mit einem Ausschluss-Molekulargewicht von mindestens 30 kDa erhaltenen Filtrats vorliegt.

8. Verfahren nach einem der Ansprüche 6 und 7, wobei das Verfahren für die Behandlung von protein- und/oder prioneninfiziertem Abfall bestimmt ist.

9. Verfahren nach einem der Ansprüche 6-8, wobei das Verfahren für die Reinigung von protein- und/oder prioneninfizierten Textilien bestimmt ist.

10. Verfahren nach einem der Ansprüche 6-9, wobei der Prionenproteinabbau und/oder der Abbau von Proteinablagerungen in Lebensmitteln stattfindet.

11. Verfahren nach einem der Ansprüche 6-10, wobei der Prionenproteinabbau und/oder der Abbau von Proteinablagerungen auf der Oberfläche eines festen Materials stattfindet.

## Revendications

1. Utilisation ex vivo d'un surnageant de culture d'*A*. *pernix* comprenant la sérine protéase d'*A*. *pernix,* ou un fragment actif de celle-ci, ou une protéine présentant une identité d'au moins 80 % avec la sérine protéase d'*A*. *pernix,* ou un fragment actif de celle-ci, pour la dégradation de dépôts de protéine prion et/ou de protéine.

2. Utilisation selon la revendication 1, dans laquelle ledit surnageant de culture d'*A. pernix* est purifié par ultrafiltration avec un poids moléculaire seuil d'au moins 30 kDa.

3. Utilisation selon l'une quelconque des revendications précédentes, dans laquelle ladite dégradation desdits dépôts de protéine prion et/ou de protéine a lieu durant la stérilisation d'un équipement chirurgical.

4. Utilisation selon l'une quelconque des revendications précédentes, dans laquelle ladite dégradation desdits dépôts de protéine prion et/ou de protéine a lieu durant le nettoyage de textiles.

5. Utilisation selon l'une quelconque des revendications précédentes, dans laquelle ladite dégradation desdits dépôts de protéine prion et/ou de protéine a lieu dans la nourriture.

6. Procédé *ex vivo* de dégradation de dépôts de protéine prion et/ou de protéine comprenant l'incubation des dépôts de protéine prion et/ou de protéine avec un surnageant de culture d'*A*. *pernix* comprenant la sérine protéase d'*A. pernix,* ou un fragment actif de celle-ci, ou une protéine présentant une identité d'au moins 80 % avec la sérine protéase d'*A. pernix*, ou un fragment actif de celle-ci.

7. Procédé selon la revendication 6, dans lequel le surnageant de culture d'*A. pernix* se trouve sous la forme d'un filtrat obtenu de l'ultrafiltration d'un surnageant de culture d'*A. pernix* avec un poids moléculaire seuil d'au moins 30 kDa.

8. Procédé selon l'une quelconque des revendications 6 et 7, dans lequel ledit procédé est destiné au traitement de déchets infectés par une protéine et/ou un prion.

9. Procédé selon l'une quelconque des revendications 6 à 8, dans lequel ledit procédé est destiné au nettoyage de textiles infectés par une protéine et/ou un prion.

10. Procédé selon l'une quelconque des revendications 6 à 9, dans lequel ladite dégradation de la protéine prion et/ou ladite dégradation du dépôt de protéine ont lieu dans la nourriture.

11. Procédé selon l'une quelconque des revendications 6 à 10, dans lequel ladite dégradation de la protéine prion et/ou ladite dégradation des dépôts de protéine ont lieu sur la surface d'un matériau solide.
